(19) Europäisches Patentamt
European Patent Office
Office européen des brevets

(11) **EP 2 925 280 B1**

(12) **EUROPEAN PATENT SPECIFICATION**

(45) Date of publication and mention
of the grant of the patent:
**01.08.2018 Bulletin 2018/31**

(21) Application number: **13795742.9**

(22) Date of filing: **25.11.2013**

(51) Int Cl.:
*A61Q 11/00* (2006.01)     *A61K 8/02* (2006.01)
*A61K 8/19* (2006.01)     *A61K 8/73* (2006.01)
*A61K 8/97* (2017.01)

(86) International application number:
**PCT/EP2013/074578**

(87) International publication number:
**WO 2014/082951 (05.06.2014 Gazette 2014/23)**

(54) **ORAL CARE COMPOSITIONS**

MUNDPFLEGEMITTEL

COMPOSITIONS DE PROTECTION ORALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.11.2012 EP 12194410**

(43) Date of publication of application:
**07.10.2015 Bulletin 2015/41**

(73) Proprietors:
• **Unilever PLC**
**London, Greater London EC4Y 0DY (GB)**
Designated Contracting States:
**CY GB IE MT**
• **UNILEVER N.V.**
**3013 AL Rotterdam (NL)**
Designated Contracting States:
**AL AT BE BG CH CZ DE DK EE ES FI FR GR HR HU IS IT LI LT LU LV MC MK NL NO PL PT RO RS SE SI SK SM TR**

(72) Inventors:
• **ASHCROFT, Alexander, Thomas**
**Bebington**
**Wirral**
**Merseyside CH63 3JW (GB)**
• **BRENNAN, Lee, James**
**Bebington**
**Wirral**
**Merseyside CH63 3JW (GB)**
• **WILSON, William, John**
**Bebington**
**Wirral**
**Merseyside CH63 3JW (GB)**

(74) Representative: **Tansley, Sally Elizabeth**
**Unilever PLC**
**Unilever Patent Group**
**Colworth House**
**Sharnbrook**
**Bedford**
**Bedfordshire MK44 1LQ (GB)**

(56) References cited:
WO-A1-2009/102024    WO-A1-2012/052306
US-A- 5 041 280    US-A1- 2004 086 626
US-A1- 2007 166 242    US-A1- 2011 189 109

• **PICKLES M J ET AL: "In vitro efficacy of a whitening toothpaste containing calcium carbonate andperlite", INTERNATIONAL DENTAL JOURNAL, BUTTERWORTH AND CO., LTD, GB, vol. 55, no. 3 Suppl. 1, 1 January 2005 (2005-01-01), pages 197-202, XP009096690, ISSN: 0020-6539**

Remarks:
The file contains technical information submitted after the application was filed and not included in this specification

## Description

### Field of the Invention

The present invention is concerned with oral care compositions. More particularly, the present invention is concerned with oral care compositions containing activated citrus fibre and particulate calcium carbonate abrasive.

### Background of the Invention

Abrasives for use in oral care compositions such as dentifrices, are required to be effective in removing extrinsic stains, dental plaque and food debris which builds up on the pellicle on the surface of teeth.

In general, the efficiency of physical removal of stain, plaque and food debris can be increased by using an abrasive having increased abrasivity, or by increasing the level of abrasive incorporated into the composition. However, both these approaches also increase the risk that tooth surfaces may be damaged.

Accordingly, there is a continuing need for oral care compositions that demonstrate satisfactory levels of cleaning, yet are not unduly abrasive and damaging to the teeth.

The present inventors have found that the cleaning efficiency of an oral care composition comprising particulate calcium carbonate abrasive may be surprisingly enhanced by the inclusion of activated citrus fibre.

Citrus fibres and their uses for structuring of foodstuffs and personal care compositions are described in US2004/0086626 and US2009/269376. The compatibility of an activated citrus fibre structured liquid detergent composition with cleaning and care enzymes is described in WO2012/052306. Its use with cationic deposition polymer for anti-dandruff shampoo is disclosed in WO2012/019934.

US 7,981,855 discloses detergent liquid surfactant compositions comprising up to 15 wt% surfactant, including at least 1% anionic surfactant, up to 2 wt% bacterial cellulose (preferably microfibrous cellulose) and from 0.001 to 5 wt% citrus fibres. US-A-2007/0166242, US-A-2011/0189109 and US-A-5,041,280 disclose oral care compositions comprising calcium carbonate abrasive and different fibres for structuring said compositions.

### Summary of the Invention

The present invention provides an oral care composition suitable for cleaning the surfaces of the oral cavity, the composition comprising:

an aqueous continuous phase including at least 1% by weight (based on the total weight of the composition) of one or more polyhydric alcohols;

from 1 to 70% by weight (based on the total weight of the composition) of particulate calcium carbonate abrasive in which the particulate calcium carbonate abrasive has an average particle size (median ESD) ranging from 1 to 15 microns and which is dispersed in the aqueous continuous phase, characterised in that the aqueous continuous phase is structured with a matrix of activated citrus fibre particles.

### Detailed Description of the Invention

Particulate calcium carbonate abrasive

The composition of the invention comprises from 1 to 70% by weight (based on the total weight of the composition) of particulate calcium carbonate abrasive which is dispersed in the aqueous continuous phase.

Preferred particulate calcium carbonate abrasives for use in the invention may be characterised by the specific shape and size of their constituent primary particles.

By "primary particles" is meant individual particles, defined as the smallest discrete particles that can be seen by electron microscopy analysis (such as, for example, individual crystals).

The primary particles may associate under certain conditions to form larger secondary structures such as aggregates or agglomerates.

For the purposes of the present invention, a suitable source of particulate calcium carbonate includes crystalline calcium carbonates.

The term "crystalline" (in the context of particulate calcium carbonate) generally means a particulate calcium carbonate in which at least 50% by weight, preferably at least 75% by weight, more preferably at least 90% by weight, most preferably more than 95% by weight and ideally more than 99% by weight of the calcium carbonate particles are in the form of crystals.

The term "crystal" means an essentially fully dense solid composed of atoms arranged in an orderly repetitive array bounded by plane surfaces which are the external expression of internal structure. Crystals may be identified and characterised by standard techniques known to those skilled in the art such as X-ray diffraction.

Crystalline calcium carbonate abrasives for use in the invention are available naturally (through the extraction and processing of natural ores) or synthetically (through chemical precipitation). There are three main crystalline morphologies, calcite, aragonite, and less commonly found, vaterite. The vaterite form of calcium carbonate is metastable and irreversibly transforms into calcite and aragonite. There are many different polymorphs (crystal habits) for each of these crystalline forms. The calcite crystalline morphology is the most commonly used crystal form of calcium carbonate. Over 300 crystalline forms of calcite have been reported in the literature.

Particle (e.g. crystal) size may be determined by standard techniques known to those skilled in the art such as sedimentation. Particle size values obtained from sedimentation techniques are normally expressed in terms of the equivalent spherical diameter (ESD), i.e. the diameter of a notional sphere having the same volume as the particle. The ESD value may be calculated based on the sedimentation rate of the particle in question as defined by Stokes' Law (Micromeritics SediGraph® 5100 Particle Size Analysis System Operator's Manual, V2.03, 1990). The sedimentation rate is measured using a finely collimated beam of low energy X-rays which pass through the sample cell to a detector. For a sample population of particles, the distribution of particle mass at various points in the cell affects the number of X-ray pulses reaching the detector. This X-ray pulse count is used to derive the particle size distribution expressed as the percent mass at given particle diameters. The median value of the ESD ("median ESD") which can be derived from this distribution (i.e. the particular ESD value on the cumulative mass distribution curve at which 50 percent mass of the population has a higher ESD and 50 percent mass of the population has a lower ESD) is usually quoted as the average particle size of the sample population.

Good cleaning performance has been obtained with crystalline calcium carbonates in which at least 50% by weight, preferably at least 80% by weight of the crystals are rhombohedral calcite crystals with an average particle size ("median ESD" as described above) from 1 to 15 microns.

Suitable sources of crystalline calcium carbonate of the size and shape defined above include natural ground calcium carbonate abrasives, generally obtainable from mining and mechanical grinding of sedimentary rocks such as limestone or chalk, or metamorphic rocks such as marble. Natural calcium carbonate is usually of the calcitic polymorph, with a crystal morphology which may typically be characterised as rhombohedral.

Preferred natural ground calcium carbonate abrasives of the type defined above are selected from ground limestone, chalk or marble, optionally refined or partially refined to remove impurities, and having an average particle size ("median ESD" as described above) ranging from 1 to 15 microns, more preferably ranging from 1.5 to 10 microns, most preferably from 2.5 to 9 microns. Commercially available examples include those materials sold by the company OMYA™ AG under the names OMYACARB™ 2-AV and OMYACARB™ 5-AV. These are fine ground high purity white marble abrasives having an average particle size ("median ESD" as described above) of about 3 and about 5 microns respectively.

In one aspect of the invention good cleaning results without excessive abrasion have been obtained with crystalline calcium carbonates in which the individual crystals have a scalenohedral morphology.

References to a "scalenohedral crystal morphology" (in the context of crystalline calcium carbonates) generally denote a crystalline calcium carbonate in which at least 50% by weight, preferably at least 75% by weight, more preferably at least 90% by weight, most preferably more than 95% by weight and ideally more than 99% by weight of the crystals are scalenohedral crystals. Scalenohedral crystals have a set of faces that are inclined to the vertical or "c"-axis direction, each of which are scalene triangles (i.e. triangles whose three sides are unequal in length). For the purposes of the present invention, preferred scalenohedral crystals have a trigonal scalenohedral shape, with twelve scalene triangle faces. Crystal shape may be determined by standard techniques known to those skilled in the art such as scanning electron microscopy (SEM). SEM is an imaging and analysis technique based on the detection of electrons and X-rays that are emitted from a material when irradiated by a scanning electron beam. Imaging allows the user to distinguish between primary particles and aggregates or agglomerates.

For the purposes of the present invention, preferred scalenohedral calcium carbonate crystals may be further characterised as having a BET specific surface area higher than or equal to $0.1 \ m^2/g$, preferably higher than or equal to $1 \ m^2/g$, more preferably higher than or equal to $3 \ m^2/g$ and most preferably higher than or equal to $4 \ m^2/g$. The calcium carbonate particles according to the invention generally have a BET specific surface area lower than or equal to $30 \ m^2/g$, preferably lower than or equal to $25 \ m^2/g$, more preferably lower than or equal to $20 \ m^2/g$, and most preferably lower than or equal to $15 \ m^2/g$. The BET specific surface area is usually measured according to the standard ISO 9277 which specifies the determination of the overall specific external and internal surface area of disperse or porous solids by measuring the amount of physically adsorbed gas according to the Brunauer, Emmett and Teller (BET) method.

As indicated above, primary particles (such as individual crystals) may associate under certain conditions to form larger secondary structures such as aggregates or agglomerates. Specifically, the scalenohedral calcium carbonate crystals described above may associate under certain conditions to form secondary structures. The size of these secondary structures is generally around Dp (as defined above) x 10, and may be determined by standard techniques known to those skilled in the art such as sedimentation. Particle size values obtained from sedimentation techniques are also normally expressed in terms of the equivalent spherical diameter (ESD), i.e. the diameter of a notional sphere having the same volume as the particle. The ESD value may be calculated based on the sedimentation rate of the particle in question as defined by Stokes' Law (Micromeritics SediGraph® 5100 Particle Size Analysis System Operator's Manual, V2.03, 1990). The sedimentation rate is measured using a finely collimated beam of low energy X-rays which pass through the sample cell to a detector. For a sample population of particles, the distribution of particle mass at various points in the cell affects the number of X-ray pulses reaching the detector. This X-ray pulse count is used to derive the particle size distribution expressed as the percent mass at given particle diameters. The median value of the ESD which can be derived from this distribution (i.e. the particular ESD value on the cumulative mass distribution curve at which 50 percent mass of the population has a higher ESD and 50 percent mass of the population has a lower ESD) is usually quoted as the average particle size of the sample population. Preferred scalenohedral calcium carbonate crystals for use in the invention may form secondary structures such as rosettes, clusters or "starburst" structures (i.e a multiplicity of primary particles emanating from a central core) with an average particle size (median ESD determined by sedimentation) ranging from about 2 to 3 microns.

Crystalline forms of calcium carbonate having scalenohedral crystal morphology (as described above) and suitable for use in the invention are available naturally, or may be produced by precipitation production technology. Typically, precipitated calcium carbonate is prepared by exposing calcium hydroxide slurry to a carbonation reaction. Control of the specific solution environment during the nucleation and growth of calcium carbonate governs the size and the shape of the crystals in the resulting precipitated calcium carbonate product.

A commercially available source of particulate calcium carbonate suitable for use in the invention is SOCAL® S2E Precipitated Calcium Carbonate (PCC), ex Solvay S.A.

Mixtures of any of the above described materials may also be used.

The relative amount of scalenohedral calcium carbonate to be incorporated into such a composition will depend to some extent on the type of composition used and its typical mode of use (for example typical product dosage and the amount of time it stays in contact with the affected site).

In dentifrices for use in the invention, for example, the amount of scalenohedral calcium carbonate may generally range from 1 to 50%, preferably from 10 to 40%, more preferably from 20 to 40%, by total weight scalenohedral calcium carbonate based on the total weight of the dentifrice.

Other types of calcium carbonate abrasive may also be suitable, depending on the particular balance of cleaning and abrasion required from a consumer perspective.

Citrus fibre

The aqueous continuous phase of the composition of the invention is structured with a matrix of activated citrus fibre particles.

Citrus fibre particles are obtainable from the albedo of citrus fruits. The albedo is generally defined as the white, inner part of the peel, consisting of spongy tissue, containing a matrix of thin walled cells with intercellular air spaces and a few vascular bundles. According to a typical preparation procedure, citrus fibre particles are prepared by taking the insoluble plant cell wall material from lemons and limes, after the juice and oil have been extracted, and then milling, drying and sieving this material. The citrus fibre particles may be further purified with extra washing steps before drying, milling and sieving. Particle size generally ranges from 10 to 500 micron. The particles do not dissolve in water due to their high content of cellulose and hemicellulose but they show excellent swelling properties.

Citrus fibre is termed 'fibre' because of its dietary fibre composition (i.e. the cellulose, hemicellulose and pectin from cell walls) rather than the morphology of its particles. Microscopy studies on citrus fibre particles typically show a heterogeneous mixture of particles having various sizes and shapes. Whilst some of the particles are fibrous in nature, many are spherical. Generally, the majority of the material consists of aggregated lumps of cell walls and cell wall debris. However, a number of tube-like structures with an open diameter of about 10 microns, often arranged in clusters, can be identified. These are water transport channels that are mainly located in the peel of citrus fruits, and termed xylem vessels. The xylem vessels consist of stacks of dead cells, joined together to form relatively long tubes, 200 to 300 microns long. The outsides of the tubes are reinforced by lignin, which is often laid down in rings or helices, preventing the tubes from collapse due to the capillary forces acting on the tube walls during water transport.

A preferred source of citrus fibre particles for use in the composition of the invention is Herbacel AQ Plus citrus fibre. This material is commercially available as a dried powder from Herbafood Ingredients GmbH, Germany. It has a total (soluble and insoluble) fibre content of greater than 80% and soluble fibre content of greater than 20%. It is supplied as a fine dried powder and has a water binding capacity of about 20 kg water per kg of powder.

The term "activation" in the context of the present invention generally means a process in which the citrus fibre particles are hydrated and dispersed. This process swells and breaks up the particles and enables them to form a structuring matrix when incorporated into aqueous media (such as the aqueous continuous phase of the composition of the invention).

In a typical activation process, dried powdered citrus fibre is activated by high shear dispersion at a low concentration in water to form a premix. It is advantageous to include a preservative into the premix.

The shear should not be high enough to lead to defibrillation. If a high-pressure homogeniser is used it should be operated between 200 and 600 bar. The more shear that is applied the less dense the resulting particles. Whilst the morphology is changed by the high shear, the aggregate size appears not to be changed. The fibres break down and then fill the water phase. The shear also rubs loose the outer parts of the cell walls and these are able to form a matrix that structures the water outside of the volume of the original fibre.

An activated citrus fibre premix may alternatively be made by milling using a high shear mixer, such as a Silverson. The premix may be passed through several sequential high-shear stages in order to ensure full hydration and dispersal of the citrus fibre.

The premix may be left to hydrate further (age) after the high shear dispersal. The activated premix is preferably used fresh.

The level of activated citrus fibre in a premix preferably lies in the range 1 to 5%, more preferably 1.5 to 2.5% by total weight citrus fibre (on a dry basis) based on the total weight of the premix. The concentration of citrus fibre in the pre-mix depends on the ability of the equipment to deal with the higher viscosity due to higher concentrations. Preferably the amount of water in the premix is at least 20 times greater than the amount of citrus fibres, more preferably at least 25 times even as much as 50 times. It is advantageous that there is excess water in order to hydrate the citrus fibre fully.

Preferred premixes have a measured yield stress of at least 70 Pa measured using an Anton Paar serrated cup and bob geometry at 25°C.

The amount of citrus fibre in the final oral care composition according to the invention is suitably at least 0.025%, and generally ranges from 0.05 to 5%, preferably from 0.1 to 4%, more preferably from 0.2 to 3% by total weight citrus fibre (on a dry basis) based on the total weight of the composition.

Product Form

The composition of the invention comprises an aqueous continuous phase including at least 1% by weight (based on the total weight of the composition) of one or more polyhydric alcohols.

Examples of suitable product forms for compositions of the invention include dentifrices, mouthwashes, chewing gums, lozenges, bioadhesive patches or strips, mouth sprays and liquid formulations, such as paints or lacquers, which are designed to be applied directly to the tooth surface using an applicator such as a brush or sponge tip applicator.

Preferred product forms for compositions of the invention are those which are suitable for brushing and/or rinsing the surfaces of the oral cavity.

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. In general, a dentifrice may take various physical forms such as solid, semi-solid or liquid (or a combination thereof). For the purposes of this invention the dentifrice is preferably in the form of a paste or a gel (or a combination thereof).

A dentifrice composition according to the invention will usually contain, as the aqueous continuous phase, a mixture of water and polyhydric alcohol in various relative amounts, with the amount of water generally ranging from 10 to 45% by weight (based on the total weight of the dentifrice) and the amount of polyhydric alcohol generally ranging from 30 to 70% by weight (based on the total weight of the dentifrice).

Typical polyhydric alcohols for use in a dentifrice composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

A dentifrice composition according to the invention will generally contain further ingredients to enhance performance and/or consumer acceptability.

For example, the dentifrice may comprise additional abrasive materials (in addition to the particulate calcium carbonate abrasives described above). Such other abrasive materials will generally be present in an amount of from 0.5 to 75% by weight based on the total weight of the dentifrice. Suitable other abrasive materials include abrasive silicas , other calcium carbonates (which are different to the particulate calcium carbonate abrasives described above), dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Furthermore, the dentifrice may also contain additional binders or thickening agents (in addition to the activated citrus fibre particles described above). Such other binders or thickening agents will generally be present in an amount of in an amount of from 0.5 to 10% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Furthermore, the dentifrice will usually contain a surfactant in an amount of from 0.2 to 5% by weight based on the total weight of the dentifrice. Suitable surfactants include anionic surfactants, such as the sodium, magnesium, ammonium or ethanolamine salts of $C_8$ to $C_{18}$ alkyl sulphates (for example sodium lauryl sulphate), $C_8$ to $C_{18}$ alkyl sulphosuccinates (for example dioctyl sodium sulphosuccinate), $C_8$ to $C_{18}$ alkyl sulphoacetates (such as sodium lauryl sulphoacetate), $C_8$ to $C_{18}$ alkyl sarcosinates (such as sodium lauryl sarcosinate), $C_8$ to $C_{18}$ alkyl phosphates (which can optionally comprise up to 10 ethylene oxide and/or propylene oxide units) and sulphated monoglycerides. Other suitable surfactants include nonionic surfactants, such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Other suitable surfactants include amphoteric surfactants, such as betaines or sulphobetaines. Mixtures of any of the above described materials may also be used.

Another example of a preferred type of product form in the context of the present invention is a mouthwash. The term "mouthwash" generally denotes liquid formulations which are used to rinse the surfaces of the oral cavity and provide the user with a sensation of oral cleanliness and refreshment. The mouthwash is an oral composition that is not intentionally swallowed for purposes of systemic administration of therapeutic agents, but is applied to the oral cavity, used to treat the oral cavity and then expectorated.

A mouthwash composition according to the invention will usually contain, as the aqueous continuous phase, a mixture of water and polyhydric alcohol in various relative amounts, with the amount of water generally ranging from 70 to 95% by weight (based on the total weight of the mouthwash), and the amount of polyhydric alcohol generally ranging from 5 to 20% by weight (based on the total weight of the mouthwash).

Typical polyhydric alcohols for use in a mouthwash composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof.

A mouthwash composition according to the invention will also generally contain further ingredients to enhance performance and/or consumer acceptability, such as the surfactants mentioned above for dentifrices. The amount of surfactant generally ranges from 0.1 to 5% by weight based on the total weight of the mouthwash.

Compositions of the present invention (such as in particular dentifrices or mouthwashes) may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

Therefore the invention also provides a composition for use in a method for cleaning the surfaces of the oral cavity comprising the following steps:

a) topically applying the composition according to the invention to the oral cavity surfaces to be cleaned;

b) agitating the composition over the oral cavity surfaces by brushing.

The invention is further illustrated with reference to the following, non-limiting Examples.

## Examples

An activated citrus fibre premix was prepared, having ingredients as shown in Table 1 below.

**Table 1**

| Material | % as supplied | Weight (g) |
|---|---|---|
| Demineralised water | 97.92 | 1958.4 |
| Proxel GXL | 0.08 | 1.6 |
| Herbacel AQ Plus citrus fibre[1] | 2.00 | 40.0 |
| [1] Powdered dried citrus fibre (ex Herbafood Ingredients GmbH, Germany) | | |

The demineralised water was stirred using an agitator stirrer with overhead drive operated at 160 rpm. The Proxel GXL preservative was added. Then Herbacel AQ Plus citrus fibre was added gradually to ensure no clumping. Stirring was continued for a further 15 minutes to allow the fibres to swell sufficiently prior to the activation stage. The activation stage was carried out by high pressure homogenisation (HPH) at 500 barg.

Three dentifrice formulations were prepared using the activated citrus fibre premix described above. Sufficient freshly made premix was added to a mixer to give the required level of activated citrus fibre in the finished composition and it was milled for 10 minutes. The remaining ingredients of the dentifrice formulations were then added. A homogenous dispersion was made using an in-line Silverson (L5T), operating at 9000 rpm with a residence time of 2 minutes.

The ingredients of the resulting dentifrice formulations are shown in Tables 2 and 3 below. The level of citrus fibre in the formulation is quoted on a dry basis. Numbered Examples represent formulations according to the invention. Lettered Examples are comparative examples (not according to the invention).

**Table 2**

| Ingredient | Example A | Ex. 1 | Ex. 2 | Ex. 3 | Ex. B | Ex. C |
|---|---|---|---|---|---|---|
| Water | to 100 | to 100 | to 100 | to 100 | to 100 | to 100 |
| Sorbitol | 40 | 40 | 40 | 40 | 21 | 21 |
| Sodium lauryl sulphate | 1.5 | 1.5 | 1.5 | 1.5 | 1.6 | 1.6 |
| Herbacel AQ Plus citrus fibre[1] | 2.5 | 2.5 | 1.5 | 1.5 | - | - |
| OMYACARB® 5-AV[2] | - | 20 | 40 | | 40 | |
| Socal® S2E[3] | | | | 40 | | 40 |
| Thickening silica | | | | | 2.4 | 2.4 |
| Flavour | | | | | 1.5 | 1.5 |
| Phosphate buffers | | | | | 1.1 | 1.1 |
| Sodium Carboxymethylc ellulose | | | | | 0.7 | 0.7 |

[2] Ground calcium carbonate ex OMYA™ AG, which is fine ground natural marble from Avenza, Italy, having a rhombohedral particle shape and an average particle size (median ESD) of about 5 microns.
[3] Precipitated calcium carbonate ex Solvay Inc., consisting of scalenohedral calcite crystals. The crystals have an average particle size (median ESD) of about 2.7 microns.

**Table 3**

| Ingredient | Example D | Ex. 4 | Ex. 5 | Ex. 6 | Ex. E | Ex. F |
|---|---|---|---|---|---|---|
| Water | to 100 | to 100 | to 100 | to 100 | to 100 | to 100 |
| Sorbitol | 21 | 40 | 40 | 40 | 40 | 21 |
| Sodium lauryl sulphate | 1.6 | 1.5 | 1.5 | 1.5 | 1.5 | 1.6 |
| Herbacel AQ Plus citrus fibre[1] | - | 1.5 | 2.5 | 1.5 | 1.5 | - |
| OMYACARB ® 5-AV[2] | 20 | 20 | | | | |
| Socal® S2E[3] | | | 20 | 20 | | |

(continued)

| Ingredient | Example D | Ex. 4 | Ex. 5 | Ex. 6 | Ex. E | Ex. F |
|---|---|---|---|---|---|---|
| ViCALity® ALBAFIL® PCC[4] | | | | | 40 | 40 |
| Thickening silica | 2.4 | | | | | 2.4 |
| Flavour | 1.5 | | | | | 1.5 |
| Phosphate buffers | 1.1 | | | | | 1.1 |
| Sodium Carboxymeth ylcellulose | 0.7 | | | | | 0.7 |
| [4] Precipitated calcium carbonate ex Speciality Minerals Inc., consisting of prismatic calcite crystals. The crystals have an average particle size (median ESD) of about 0.7 microns. | | | | | | |

The PCR was measured for each of the above formulations. PCR was measured using a method based on that described by Pickles et al. (International Dental Journal 55 (2005), pp. 197-202). This model uses enamel slabs cut from bovine central incisors, embedded in methacrylate resin. The enamel surfaces are smoothed by hand using an alumina paste on a glass block and lightly acid etched in order to facilitate stain accumulation and adherence. The enamel blocks are placed in an incubator set at a constant temperature of 50°C and slowly rotated to alternate between immersion in a staining broth (consisting of tea, coffee and mucin) and air drying. The broth is changed daily and after five days the slabs are removed and washed with distilled water to remove any loose debris. The stained slabs are then brushed in a mechanical brushing machine with distilled water to remove any loosely adhered stain. They are then dried and the L* values ($L^*_{stained}$) from the CIE L*a*b* colour system are measured with a chroma meter in L*a*b* mode. To assess cleaning performance, the stained specimens are then mounted in the mechanical brushing machine and the required load applied to each brush. The test composition is dispersed in an aqueous diluent to form a slurry (typically 38.5g test composition and 61.5g of distilled water) and the stained specimens brushed for a set number of brush strokes with 10ml of slurry. After brushing, the enamel slabs are rinsed with distilled water, dried and the L* values ($L^*_{brushed}$) are re-measured. In the final stage, all traces of stain are removed from the enamel slabs using flour of pumice, on a soft cloth using a grinder/polisher. The enamel slabs are then rinsed with distilled water, dried and the L* values ($L^*_{pumiced}$) are measured and recorded. The percentage of the stain removed by the test composition compared to full removal by pumice (hereinafter referred to as the pellicle cleaning ratio or PCR) may be calculated using the following equation:

$$PCR = [(L^*_{brushed})-(L^*_{stained})/ (L^*_{pumiced})-(L^*_{stained})] \times 100$$

The PCR results after 800 brush strokes are shown in Table 4 below.

**Table 4**

| Test Formulation | Mean 800 (s.d.) |
|---|---|
| Example A | 8.22 (3.85) |
| Example 1 | 59.15 (5.31) |
| Example 2 | 64.99 (9.56) |
| Example 3 | 47.26 (6.53) |
| Example B | 49.64 (4.57) |
| Example C | 43.89 (8.26) |
| Example D | 51.43 (9.11) |
| Example 4 | 64.61 (5.97) |
| Example 5 | 44.48 (4.13) |
| Example 6 | 70.24 (8.50) |
| Example E | 35.47 (7.36) |
| Example F | 36.27 (4.25) |

It can be seen from these results that the activated citrus fibre (Example A) has little or no cleaning ability on its own. However, when it is combined with calcium carbonate particles of the claimed particle size (Examples 1 - 3 and 4 - 6), the cleaning performance is surprisingly enhanced, versus the control examples, with the combinations in Examples 1, 2, 4 and 6 being particularly effective. However, it can also be seen that the small particle size calcium carbonate used in example E shows no cleaning enhancement with citrus fibre when compared with comparative example F.

**Claims**

1. An oral care composition suitable for cleaning the surfaces of the oral cavity, the composition comprising:

    an aqueous continuous phase including at least 1 % by weight based on the total weight of the composition of one or more polyhydric alcohols;
    from 1 to 70% by weight based on the total weight of the composition of particulate calcium carbonate abrasive in which the particulate calcium carbonate abrasive has an average particle size (median ESD) ranging from 1 to 15 microns and which is dispersed in the aqueous continuous phase,
    **characterised in that** the aqueous continuous phase is structured with a matrix of activated citrus fibre particles.

2. An oral care composition according to claim 1, in which the particulate calcium carbonate abrasive has an average particle size (median ESD) ranging from 2.5 to 9 microns.

3. An oral care composition according to any preceding claim in which the particulate calcium carbonate abrasive is selected from ground limestone, chalk or marble.

4. An oral care composition according to claim 1 or claim 2, in which the particulate calcium carbonate abrasive is selected from crystalline calcium carbonates in which the individual crystals have a scalenohedral morphology.

5. An oral care composition according to any preceding claim, which is in the form of a dentifrice and which contains from 10 to 45% by weight water based on the total weight of the dentifrice and from 30 to 70% by weight polyhydric alcohol based on the total weight of the dentifrice.

6. A dentifrice according to claim 5, which further comprises a surfactant in an amount of from 0.2 to 5% by weight based on the total weight of the dentifrice.

7. An oral care composition according to any preceding claim, in which the amount of citrus fibre ranges from 0.2 to 3% by total weight citrus fibre on a dry basis based on the total weight of the composition.

8. A composition for use in a method for cleaning the surfaces of the oral cavity comprising the following steps:

    a) topically applying the composition according to any one of claims 1 to 7 to the oral cavity surfaces to be cleaned;
    b) agitating the composition over the oral cavity surfaces by brushing.

**Patentansprüche**

1. Mundpflegezusammensetzung, die zum Reinigen der Oberflächen der Mundhöhle geeignet ist, wobei die Zusammensetzung umfasst:

    eine kontinuierliche wässrige Phase, die zu mindestens 1 Gewichts-%, bezogen auf das Gesamtgewicht der Zusammensetzung, einen oder mehrere mehrwertige Alkohole enthält,
    von 1 bis 70 Gewichts-%, bezogen auf das Gesamtgewicht der Zusammensetzung, teilchenförmiges Calciumcarbonat-Schleifmittel, in welchem das teilchenförmige Calciumcarbonat-Schleifmittel eine durchschnittliche Teilchengröße (Median ESD) zwischen 1 und 15 Mikron aufweist und welches in der kontinuierlichen wässrigen Phase dispergiert ist, **dadurch gekennzeichnet,**
    **dass** die kontinuierliche wässrige Phase mit einer Matrix aktivierter Citrusfaserteilchen strukturiert ist.

2. Mundpflegezusammensetzung nach Anspruch 1, in welcher das teilchenförmige Calciumcarbonat-Schleifmittel eine durchschnittliche Teilchengröße (Median ESD) aufweist, die zwischen 2,5 und 9 Mikron liegt.

3. Mundpflegezusammensetzung nach irgendeinem vorhergehenden Anspruch, in welcher das teilchenförmige Calciumcarbonat-Schleifmittel unter gemahlenem(r) Kalkstein, Kreide oder Marmor ausgewählt ist.

4. Mundpflegezusammensetzung nach Anspruch 1 oder Anspruch 2, in welcher das teilchenförmige Calciumcarbonat-Schleifmittel unter kristallinen Calciumcarbonaten ausgewählt ist, in welchen die individuellen Kristalle eine skalenoedrische Morphologie aufweisen.

5. Mundpflegezusammensetzung nach einem vorhergehenden Anspruch, welche in Form eines Zahnpflegemittels vorliegt und welche von 10 bis 45 Gewichts-% Wasser, bezogen auf das Gesamtgewicht des Zahnpflegemittels, und von 30 bis 70 Gewichts-% mehrwertigen Alkohol, bezogen auf das Gesamtgewicht des Zahnpflegemittels, enthält.

6. Zahnpflegemittel nach Anspruch 5, das ferner ein Tensid in einer Menge von 0,2 bis 5 Gewichts-%, bezogen auf das Gesamtgewicht des Zahnpflegemittels, enthält.

7. Mundpflegezusammensetzung nach einem vorhergehenden Anspruch, in welcher die Menge der Citrusfaser zwischen 0,2 und 3 Gewichts-%, in Gesamtgewicht der Citrusfaser auf Trockenbasis, bezogen auf das Gesamtgewicht der Zusammensetzung, liegt.

8. Zusammensetzung zur Verwendung in einem Verfahren zum Reinigen der Oberflächen der Mundhöhle, umfassend die folgenden Schritte:

a) örtliches Auftragen der Zusammensetzung nach irgendeinem der Ansprüche 1 bis 7 auf die zu reinigenden Oberflächen der Mundhöhle,
b) Bewegen der Zusammensetzung über die Oberflächen der Mundhöhle durch Bürsten.


**Revendications**

1. Composition pour le soin oral appropriée pour nettoyer les surfaces de la cavité orale, la composition comprenant :

une phase aqueuse continue incluant au moins 1 % en masse sur la base de la masse totale de la composition d'un ou plusieurs alcools polyvalents ;
de 1 à 70 % en masse sur la base de la masse totale de la composition d'abrasif de carbonate de calcium particulaire dans laquelle l'abrasif de carbonate de calcium particulaire présente une taille moyenne de particule (ESD médian) de 1 à 15 microns et qui est dispersé dans la phase aqueuse continue, **caractérisée en ce que** la phase aqueuse continue est structurée avec une matrice de particules de fibres d'agrumes activées.

2. Composition pour le soin oral selon la revendication 1, dans laquelle l'abrasif de carbonate de calcium particulaire présente une taille moyenne de particule (ESD médian) de 2,5 à 9 microns.

3. Composition pour le soin oral selon l'une quelconque des revendications précédentes, dans laquelle l'abrasif de carbonate de calcium particulaire est choisi parmi du calcaire, de la craie ou du marbre broyé.

4. Composition pour le soin oral selon la revendication 1 ou la revendication 2, dans laquelle l'abrasif de carbonate de calcium particulaire est choisi parmi des carbonates de calcium cristallins dans lesquels les cristaux individuels présentent une morphologie scalénohédrique.

5. Composition pour le soin oral selon l'une quelconque des revendications précédentes, laquelle est dans la forme d'un dentifrice et qui contient de 10 à 45 % en masse d'eau sur la base de la masse totale du dentifrice et de 30 à 70 % en masse d'alcool polyvalent sur la base de la masse totale du dentifrice.

6. Dentifrice selon la revendication 5, qui comprend de plus un tensioactif dans une quantité de 0,2 à 5 % en masse rapporté à la masse totale du dentifrice.

7. Composition pour le soin oral selon l'une quelconque des revendications précédentes, dans laquelle la quantité de fibres d'agrumes est de 0,2 à 3 % en masse totale de fibres d'agrumes sur une base sèche rapporté à la masse totale de la composition.

**8.** Composition pour une utilisation dans un procédé pour le nettoyage des surfaces de la cavité orale comprenant les étapes suivantes :

a) d'application topique de la composition selon l'une quelconque des revendications 1 à 7 aux surfaces de cavités orales à nettoyer ;
b) d'agitation de la composition sur les surfaces de cavité orale par brossage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040086626 A
- US 2009269376 A
- WO 2012052306 A
- WO 2012019934 A
- US 7981855 B
- US 20070166242 A
- US 20110189109 A
- US 5041280 A

**Non-patent literature cited in the description**

- **STOKES' LAW.** *Micromeritics SediGraph® 5100 Particle Size Analysis System Operator's Manual,* 1990
- **PICKLES et al.** *International Dental Journal,* 2005, vol. 55, 197-202